# EUROPÄISCHE PATENTSCHRIFT

(11) **EP 2 240 402 B1**
(45) Veröffentlichungstag und Bekanntmachung des Hinweises auf die Patenterteilung: **18.01.2012**
(21) Anmeldenummer: 08869947.5
(22) Anmeldetag: 19.11.2008
(51) Int. Cl.: A61K 9/00, A61M 37/00, A61M 31/00

(54) **VERFAHREN ZUR HERSTELLUNG PORÖSER MIKROSTRUKTUREN, NACH DIESEM VERFAHREN HERGESTELLTE PORÖSE MIKROSTRUKTUREN SOWIE DEREN VERWENDUNG**
METHOD FOR PRODUCING POROUS MICROSTRUCTURES, POROUS MICROSTRUCTURES PRODUCED BY THIS METHOD AND USE THEREOF
PROCÉDÉ POUR PRODUIRE DES MICROSTRUCTURES POREUSES, STRUCTURES MICROPOREUSES OBTENUES SELON LE PROCÉDÉ ET LEUR UTILISATION

(30) Priorität: 08.01.2008 DE 102008003453
(43) Veröffentlichungstag der Anmeldung: 20.10.2010
(73) Patentinhaber: Robert Bosch GmbH, 70442 Stuttgart (DE)
(72) Erfinder: SCHOLTEN, Dick, 70188 Stuttgart (DE); PIRK, Tjalf, 71229 Leonberg (DE); STUMBER, Michael, 70825 Korntal-Muenchingen (DE); LAERMER, Franz, 71263 Weil Der Stadt (DE); REICHENBACH, Ralf, 73732 Esslingen (DE); FEYH, Ando, 71732 Tamm (DE)
(86) Internationale Anmeldenummer: PCT/EP2008/065806
(87) Internationale Veröffentlichungsnummer: WO 2009/086986

(56) Entgegenhaltungen:
- DE-A1-102006 028 916
- COHEN M H ET AL: "Microfabrication of silicon-based nanoporous particulates for medical applications" BIOMEDICAL MICRODEVICES - KLUWER ACADEMIC PUBLISHERS USA, Bd. 5, Nr. 3, 30. September 2003 (2003-09-30), Seiten 253-259, XP002551837 ISSN: 1387-2176

## Beschreibung

### Stand der Technik

Poröse Mikrostrukturen und ihre Verwendung zur Applikation von Wirkstoffen werden beschrieben in Cohen et al. "Microfabrication of Silicion-Based Nanoporous Particulates for Medical Applications", Biomedical Microdevices, Vol. 5, Seiten 253-259, 2003.

Die DE 10 2006 028 916 A1 offenbart ein Verfahren zur Herstellung poröser Partikel. Dabei ist ein Verfahrenschritt das Porosifizieren eines Halbleitersubstrats.

Die vorliegende Erfindung betrifft ein Verfahren zur Herstellung poröser Mikrostrukturen sowie poröse Mikrostrukturen, hergestellt nach diesem Verfahren sowie deren Verwendung.

Poröses Silizium weist durch eine große innere Oberfläche zahlreiche Verwendungsmöglichkeiten in der Medizin auf, beispielsweise als Reservoir zur Freisetzung von Medikamenten. Üblichweise wird elektrochemisch hergestelltes poröses Silizium verwendet, wobei eine poröse, schwammartige Struktur in Silizium, beispielsweise im Bereich der Oberfläche eines Siliziumwafers, erzeugt wird.

Poröse Siliziumpartikel werden gemäß den im Stand der Technik bekannten Verfahren dadurch hergestellt, dass beispielsweise auf einem Siliziumwafer großflächig eine poröse Schicht hergestellt wird, diese durch mechanische Einwirkung abgetrennt und die Partikel durch mechanisches Zerkleinern des porösen Materials, beispielsweise durch Mahlen, vereinzelt werden.

Durch das mechanische Zerkleinern entstehen Partikel mit unbestimmter Größenverteilung und Form der einzelnen Partikel. Darüber hinaus wird die Oberfläche der porösen Partikel durch die mechanische Einwirkung in der Regel stark beschädigt oder sogar zerstört. Darüber hinaus die Form der auf diese Weise hergestellten Partikel in der Regel nicht reproduzierbar, insbesondere sind auf diese Weise kaum reproduzierbar runde Partikel herstellbar.

### Offenbarung der Erfindung

Das erfindungsgemäße Verfahren zur Herstellung poröser Mikrostrukturen in einem Si-Halbleitersubstrat hat demgegenüber den Vorteil, dass poröse Siliziumstrukturen mit zuvor definierter Form und Größe herstellbar sind.

Dies wird erfindungsgemäß dadurch erreicht, dass das Verfahren die folgenden Schritte umfasst:
a) Aufbringen und Strukturieren einer Maskierungsschicht auf die äußere Oberfläche der Vorderseite eines Si-Halbleitersubstrats, wobei in der Maskierungsschicht diskrete Löcher mit einem mittleren Durchmesser im Bereich von ≥ 0,1 µm bis ≤ 500 µm ausgebildet werden,
b) Erzeugen von Ausnehmungen in einem Si-Halbleitersubstrat, wobei die Ausnehmungen jeweils eine Teilstruktur der porösen Mikrostrukturen definieren;
c) Entfernen der Maskierungsschicht;
d) Aufbringen und Strukturieren einer Opferschicht, wobei die Opferschicht diskrete durchgehende Löcher mit einem Durchmesser im Bereich von ≥ 0,1 µm bis ≤ 150 µm aufweist, die jeweils zentral in den Ausnehmungen angeordnet sind;
e) Aufbringen einer Siliziumschicht, wobei die Siliziumschicht die Ausnehmungen auffüllt und eine Schicht oberhalb der Opferschicht ausbildet;
f) Porosifizieren der Silizium-Schicht;
g) Aufbringen und Strukturieren einer weiteren Maskierungsschicht auf die porosifizierte Silizium-Schicht, wobei die weitere Maskierungsschicht die Bereiche oberhalb der Ausnehmungen bedeckt, die eine weitere Teilstruktur der porösen Mikrostrukturen ausbilden;
h) anisotropes Ätzen der porosifizierten Silizium-Schicht;
i) Entfernen der weiteren Maskierungsschicht;
j) Entfernen der Opferschicht;
k) Abtrennen oder Vereinzeln der porösen Mikrostrukturen.

Unter dem Begriff "Opferschicht" wird im Sinne der vorliegenden Erfindung eine Schicht verstanden, die in einem späteren Verfahrensschritt wieder entfernt wird.

Der Begriff "Dotieren" hat im Sinne dieser Erfindung die Bedeutung, dass der dotierte Bereich oder die dotierte Schicht eine höhere Dotierung aufweist, als der ursprüngliche Bereich bzw. ein angrenzender Bereich oder Schicht des Halbleitersubstrats. Eine Dotierung kann beispielsweise mit Bor erfolgen, insbesondere kann man mit Bor implantieren oder eine Borglasbelegung vornehmen.

Weiterhin wird durch das erfindungsgemäße Verfahren ermöglicht, runde, poröse Silizium-Mikrostrukturen mit Durchmessern von einigen Mikrometern bis zu einigen hundert Mikrometern herzustellen, sowie poröse Mikronadeln.

Ein weiterer Vorteil des erfindungsgemäßen Verfahrens kann dadurch zur Verfügung gestellt werden, dass das Verfahren nur wenige Verfahrensschritte aufweist. Vorzugsweise benötigt das erfindungsgemäße Verfahren nicht mehr als drei Maskenebenen. Dies ermöglicht die Herstellung poröser Mikrostrukturen mit geringeren Herstellungskosten.

### Zeichnungen

Ausführungsbeispiele der Erfindung sind in den Zeichnungen dargestellt und werden in der nachfolgenden Beschreibung näher erläutert. Hierbei zeigt:
- Figur 1a bis 1f: schematische Querschnittansichten der wesentlichen Herstellungsschritte poröser Mikrostrukturen in Form von Mikronadeln gemäß einer ersten Ausführungsform der vorliegenden Erfindung.
- Figur 2a bis 2f: schematische Querschnittansichten der wesentlichen Herstellungsschritte abgerundeter poröser Mikrostrukturen gemäß einer zweiten Ausführungsform der vorliegenden Erfindung.

### Beschreibung der Ausführungsbeispiele

Das erfindungsgemäße Verfahren wird zunächst beispielhaft beschrieben.

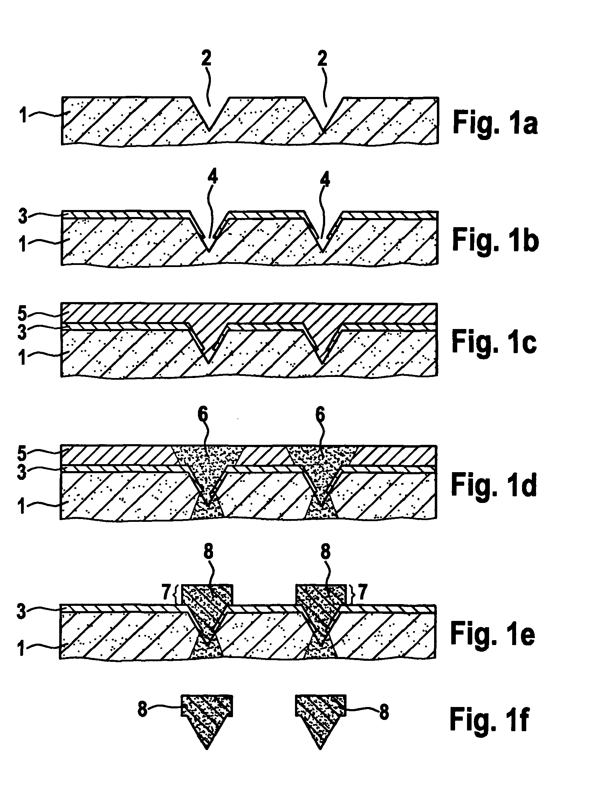

In den Figuren 1a bis 1f ist ein mögliches Herstellungsverfahren poröser Mikrostrukturen 8 in Form von Mikronadeln dargestellt.

Die Figur 1a zeigt ein Siliziumhalbleitersubstrat 1, das Ausnehmungen 2 aufweist. Zur Herstellung der Ausnehmungen 2 wurde eine Maskierungsschicht, beispielsweise ausgebildet aus SiO₂ oder Si₃N₄ auf die äußere Oberfläche der Vorderseite des Siliziumhalbleitersubstrats 1 aufgebracht und photolithographisch strukturiert. Auf das Maskierungsmaterial wurde hierzu eine Fotolackschicht, beispielsweise AZ® 1529 (Fa. AZ-Electronic Materials), aufgebracht mit einer Maske belichtet und anschließend entfernt. Anschließend wurde die Maskierungsschicht beispielsweise mittels reaktiven Ionenätzverfahren geätzt, wobei in der Maskierungsschicht diskrete Löcher entsprechend der Belichtungsmaske ausgebildet wurden.

Beispielsweise wurde zur Erzeugung der Ausnehmungen 2 eine Ätztiefe von 20 µm mit einer selbststoppenden quadratischen Maskenöffnung von 28 µm Kantenlänge in einer wässrigen KOH-Lösung bei 80°C erzeugt. Typische Ätzraten liegen im Bereich ≤ 4 µm/min. Die Ausnehmungen 2 definieren jeweils eine Teilstruktur der porösen Mikrostrukturen 8. Anschließend wurde die Maskierungsschicht entfernt.

Wie in der Figur 1b gezeigt ist, wurde auf das Si-Halbleitersubstrat 1 mit den Ausnehmungen 2 eine Opferschicht 3 aufgebracht. Hierzu wurde bei einer Temperatur von rund 1100 °C, vorzugsweise in feuchter Umgebung, während 1 bis 2 Stunden durch thermische Oxidation des Siliziumhalbleitersubstrats 1 eine Siliziumdioxidschicht einer Dicke von rund 1 µm erzeugt. Die Opferschicht 3 wurde lithographisch strukturiert. Zentral in den Ausnehmungen 2 angeordnete Löcher 4 in der Siliziumdioxidschicht mit einem Durchmesser von 8 µm wurden durch Ätzen mit einer gepufferten Flusssäurelösung enthaltend 18,5 Vol.-% HF, bezogen auf das Gesamtvolumen der Lösung, während 15 Minuten bei 25 °C geätzt.

In einem nachfolgenden Verfahrensschritt wurde eine polykristalline Siliziumschicht 5 beispielsweise mittels eines PECVD-Verfahrens aufgebracht, wie in Figur 1c gezeigt ist. Die polykristalline Siliziumschicht 5 wies eine Dicke oberhalb der Ausnehmungen im Bereich von 0,5 µm bis 5 µm auf. Anschließend wurde die Siliziumschicht 5 mittels Implantation von Bor p++ dotiert, wobei eine Dotierung mit 10¹⁹ Ladungsträgern/cm³ vorgenommen wurde. Die dotierte Siliziumschicht 5 wurde durch elektrochemisches Ätzen in einem flusssäurehaltigen Elektrolyten, mit einem Flusssäuregehalt im Bereich von 30 Vol.- % und mit einem Gehalt an Isopropanol oder Ethanol im Bereich von 10 Vol.-% bis 30 Vol.-%, bezogen auf das Gesamtvolumen des Elektrolyten, porosifiziert, wobei die Stromdichte bei 100 mA/cm² lag. Hierbei wurde eine Porosität von 40 % bis 50 % und ein Porendurchmesser von 20 nm erzeugt. Der Strom floss vorzugsweise durch die Löcher 4 in der Opferschicht 3, wodurch die Bereiche 6 in der Siliziumschicht 5 oberhalb der Ausnehmungen bevorzugt porosifiziert wurden, wie in der Figur 1d gezeigt ist.

Auf die porosifizierte Siliziumschicht 5 wurde in einem weiteren Verfahrenschritt eine Maskierungsschicht, vorzugsweise Fotolack, aufgebracht und photolithographisch strukturiert, wobei die Maskierungsschicht die porosifizierten Bereiche der Siliziumschicht 5 oberhalb der Ausnehmungen 2 bedeckte. Diese Bereiche bilden eine weitere Teilstruktur der porösen Mikrostrukturen 8 aus. Durch anschließendes anisotropes Ätzen, vorzugsweise mittels DRIE-Verfahren, wurden die Strukturen der porösen Mikrostrukturen 8 aus der porosifizierten Siliziumschicht 5 geätzt, wie in Figur 1e gezeigt ist. Die nicht bedeckten Bereiche der Siliziumschicht 5 wurden hierbei bis zum Ätzstopp auf der Siliziumoxid-Opferschicht 3 entfernt, wodurch die aus der Opferschicht 3 herausragenden Strukturen 7 der porösen Mikrostrukturen 8 ausgebildet wurden.

Anschließend wurde die Opferschicht 3 durch stromloses Gasphasenätzen unter Verwendung von HF-Dampf entfernt. Die porösen Siliziumstrukturen 8 wurden mechanisch durch Ultraschall abgelöst. Nach dem Abtrennen von dem Siliziumhalbleitersubstrat 1 wurden poröse Mikrostrukturen 8 in Form von Mikronadeln erhalten, wie in der Figur 1f gezeigt ist.

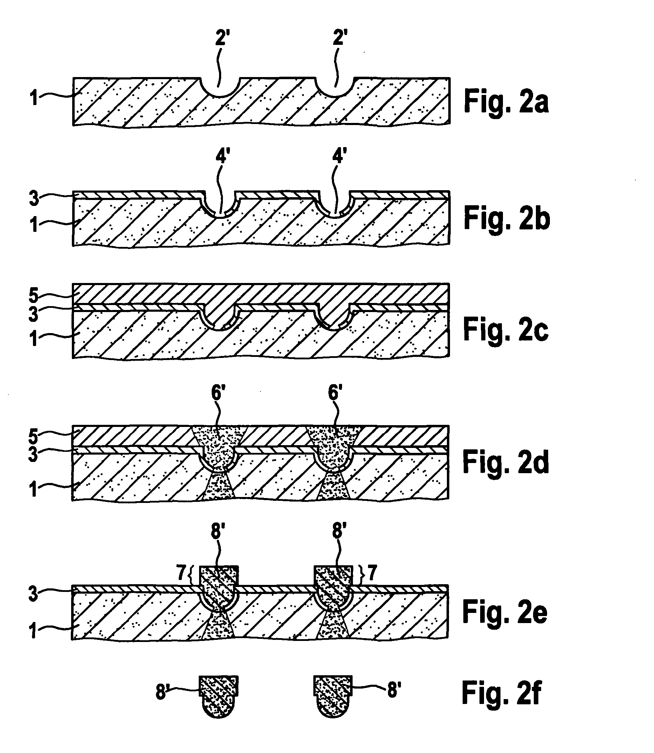

Wie in den Figuren 2a bis 2f gezeigt ist, ist das erfindungsgemäße Verfahren weiterhin verwendbar, um poröse Mikrostrukturen mit abgerundeter Form 8' zu erzeugen. In einem Si-Halbleitersubstrat 1 wurden zunächst durch isotropes Ätzen, beispielsweise mit ClF₃ abgerundete Ausnehmungen 2' erzeugt.

Zur Herstellung der Ausnehmungen 2' wurde eine Maskierungsschicht, beispielsweise ausgebildet aus SiO₂ oder Si₃N₄ auf die äußere Oberfläche der Vorderseite des Siliziumhalbleitersubstrats 1 aufgebracht und photolithographisch strukturiert. Auf das Maskierungsmaterial wurde hierzu eine Fotolackschicht, beispielsweise AZ® 1529 (Fa. AZ-Electronic Materials), aufgebracht mit einer Maske belichtet und anschließend entfernt. Anschließend wurde die Maskierungsschicht beispielsweise mittels reaktiven Ionenätzverfahren geätzt, wobei in der Maskierungsschicht diskrete Löcher entsprechend der Belichtungsmaske ausgebildet wurden.

Beispielsweise wurde zur Erzeugung der Ausnehmungen 2' durch Löcher in der Maskierungsschicht mit einem Durchmesser von 1 µm eine Ätztiefe von 30 µm mit einer Ätzrate von 10 µm/min in einer Ätzzeit von 3 Minuten mit ClF₃ erzeugt. Dieser Ätzprozess ist sehr isotrop, wodurch eine halb-runde Form erhalten werden kann. Typische Ätzraten liegen im Bereich ≤ 20 µm/min. Die Ausnehmungen 2' definieren jeweils eine Teilstruktur der porösen Mikrostrukturen 8'. Anschließend wurde die Maskierungsschicht entfernt.

Wie in der Figur 2b gezeigt ist, wurde auf das Si-Halbleitersubstrat 1 mit den Ausnehmungen 2' eine Opferschicht 3 aufgebracht. Hierzu wurde bei einer Temperatur von rund 1100 °C, vorzugsweise in feuchter Umgebung, während 1 bis 2 Stunden durch thermische Oxidation des Siliziumhalbleitersubstrats 1 eine Siliziumdioxidschicht einer Dicke von rund 1 µm erzeugt. Die Opferschicht 3 wurde lithographisch strukturiert. Zentral in den Ausnehmungen 2' angeordnete Löcher 4' in der Siliziumdioxidschicht mit einem Durchmesser von 5 µm wurden durch Ätzen mit einer gepufferten Flusssäurelösung enthaltend 18,5 Vol.-% HF, bezogen auf das Gesamtvolumen der Lösung, während 15 Minuten bei 25 °C geätzt.

In einem nachfolgenden Verfahrensschritt wurde eine polykristalline Siliziumschicht 5 beispielsweise mittels eines PECVD-Verfahrens aufgebracht, wie in Figur 2c gezeigt ist. Die polykristalline Siliziumschicht 5 wies eine Dicke oberhalb der Ausnehmungen im Bereich von 0,5 µm bis 5 µm auf. Anschließend wurde die Siliziumschicht 5 mittels Implantation von Bor p++ dotiert, wobei eine Dotierung mit 10¹⁹ Ladungsträgern/cm³ vorgenommen wurde. Die dotierte Siliziumschicht 5 wurde durch elektrochemisches Ätzen in einem flusssäurehaltigen Elektrolyten, mit einem Flusssäuregehalt im Bereich von 30 Vol.- % und mit einem Gehalt an Isopropanol oder Ethanol im Bereich von 10 Vol.-% bis 30 Vol.-%, bezogen auf das Gesamtvolumen des Elektrolyten, porosifiziert, wobei die Stromdichte bei 100 mA/cm² lag. Hierbei wurde eine Porosität von 40 % bis 50 % und ein Porendurchmesser von 20 nm erzeugt. Der Strom floss vorzugsweise durch die Löcher 4' in der Opferschicht 3, wodurch die Bereiche 6' in der Siliziumschicht 5 oberhalb der Ausnehmungen bevorzugt porosifiziert wurden, wie in der Figur 2d gezeigt ist.

Auf die porosifizierte Siliziumschicht 5 wurde in einem weiteren Verfahrenschritt eine Maskierungsschicht, vorzugsweise Fotolack, aufgebracht und photolithographisch strukturiert, wobei die Maskierungsschicht die porosifizierten Bereiche der Siliziumschicht 5 oberhalb der Ausnehmungen 2' bedeckte. Diese Bereiche bilden eine weitere Teilstruktur der porösen Mikrostrukturen 8' aus. Durch anschließendes anisotropes Ätzen, vorzugsweise mittels DRIE-Verfahren, wurden die Strukturen der porösen Mikrostrukturen 8' aus der porosifizierten Siliziumschicht 5 geätzt, wie in Figur 2e gezeigt ist. Die nicht bedeckten Bereiche der Siliziumschicht 5 wurden hierbei bis zum Ätzstopp auf der Siliziumoxid-Opferschicht 3 entfernt, wodurch die aus der Opferschicht 3 herausragenden Strukturen 7 der porösen Mikrostrukturen 8' ausgebildet wurden.

Anschließend wurde die Opferschicht 3 durch stromloses Gasphasenätzen unter Verwendung von HF-Dampf entfernt. Die porösen Siliziumstrukturen 8' wurden mechanisch durch Ultraschall abgelöst.

Nach dem Abtrennen wurden poröse Mikrostrukturen 8' mit abgerundeter Form erhalten wie in der Figur 2f gezeigt ist.

Es kann vorgesehen sein, dass vor dem Entfernen der Opferschicht 3 das Si-Halbleitersubstrat 1 für kurze Zeit in eine wässrige KOH-Lösung einer Temperatur von beispielsweise 80 °C getaucht wird, wobei die aus der Opferschicht 3 herausragenden Strukturen 7 der porosifizierten Siliziumschicht 5 durch das Ätzmittel abgerundet werden. Hierdurch können nahezu runde poröse Siliziumstrukturen beispielsweise mit einem Durchmesser von 5 µm erhalten werden.

Bevorzugte Ausgestaltungen des erfindungsgemäßen Verfahrens sind mit den nachgeordneten Patentansprüchen beansprucht und werden nachfolgend näher erläutert.

Als besonders geeignetes Silizium (Si)-Halbleitersubstrat sind Siliziumwafer verwendbar.

Gemäß dem erfindungsgemäßen Verfahren werden Ausnehmungen in dem Si-Halbleitersubstrat erzeugt, wobei die Ausnehmungen jeweils eine Teilstruktur der porösen Mikrostrukturen definieren.

Hierzu wird eine erste Maskierungsschicht auf die äußere Oberfläche der Vorderseite eines Si-Halbleitersubstrats aufgebracht und strukturiert, wobei in der ersten Maskierungsschicht diskrete Löcher mit einem mittleren Durchmesser im Bereich von ≥ 0,1 µm bis ≤ 500 µm ausgebildet werden. Im Sinne dieser Erfindung bedeutet der Begriff "Loch" einen Bereich einer Schicht, in dem die Schicht eine durchgehende Öffnung aufweist, die die äußere Oberfläche der darunter liegenden Schicht exponiert. Beispielsweise bedeutet der Begriff "Loch" einen Bereich der Maskierungsschicht, in dem die Maskierungsschicht eine durchgehende Öffnung aufweist, die die äußeren Oberfläche des Si-Halbleitersubstrats exponiert. Diese Löcher erlauben den Zugang des Ätzmittels zum Si-Halbleitersubstrat. Im Sinne dieser Erfindung bedeutet der Begriff "diskret", dass die einzelnen Löcher nicht miteinander in Verbindung stehen. Die Löcher sind bevorzugt gleichmäßig beabstandet.

Vorzugsweise verwendbare Maskierungsmaterialien sind SiO₂- oder Si₃N₄-Schichten. Die Maskierungsschicht kann auch aus anderen Substanzen ausgebildet sein, wie SiC. Im Rahmen des erfindungsgemäßen Verfahrens sind ebenfalls mittels CVD (CVD, "Chemical Vapour Deposition") aufbringbare Schichten, beispielsweise Siliziumoxid-Schichten oder geeignete Resistschichten als Maskierungsschicht verwendbar.

Die Maskierungsmaterialien, insbesondere SiO₂ oder Si₃N₄, werden bevorzugt photolitographisch strukturiert. Vorzugsweise verwendbar sind Photoresistschichten mit positiven oder negativen Belichtungseigenschaften, die anschließend bevorzugt mittels lithographischer insbesondere photolithographischer Verfahren strukturiert werden können. Geeignet sind beispielsweise flüssige Resistlacke wie Photolack. Beispielsweise verwendbar sind Photoresiste erhältlich unter der Bezeichnung AZ® 1529 (Fa. AZ-Electronic Materials). Die Photoresistschicht kann mit einer Maske, beispielsweise einer Siliziumdioxid-Schicht, belichtet und anschließend entfernt werden.

Vorzugsweise wird die Maskierungsschicht, insbesondere eine SiO₂- oder Si₃N₄-Schicht, mittels geeigneten Ätzmitteln geätzt, wobei in der Maskierungsschicht diskrete Löcher mit einem mittleren Durchmesser im Bereich von ≥ 0,1 µm bis ≤ 500 µm ausgebildet werden.

Die Löcher in der Maskierungsschicht weisen vorzugsweise eine runde Form auf. Weiterhin können die Löcher in der Maskierungsschicht eine eckige Form aufweisen, beispielsweise können die Löcher in der Maskierungsschicht quadratisch sein. Der mittlere Durchmesser der Löcher der Maskierungsschicht liegt in bevorzugten Ausführungsformen im Bereich von ≥ 0,1 µm bis ≤ 100 µm, weiter bevorzugt im Bereich von ≥ 0,1 µm bis ≤ 50 µm, vorzugsweise im Bereich von ≥ 1 µm bis ≤ 10 µm.

Die Ausnehmungen in den Si-Halbleitersubstrat können beispielsweise eine runde, abgerundete oder ovale Form aufweisen. Weiterhin können die Ausnehmungen eine eckige oder spitze Form aufweisen. In weiteren bevorzugten Ausführungsformen weisen die Ausnehmungen eine spitze Form auf, beispielsweise die Form eines Vielecks wie die eines Tetraeders. Es kann bevorzugt sein, dass sich Ausnehmungen in die Tiefe verjüngen, spitz zulaufen und/oder in einer Spitze enden. Von Vorteil ist, dass Ausführungsformen mit spitzen Ausnehmungen, jeweils eine Teilstruktur poröser Mikrostrukturen definieren können, die die Form einer Mikronadel aufweisen.

In bevorzugten Ausführungsformen weisen die Ausnehmungen eine abgerundete Form auf, insbesondere die Form einer Halbkugel oder im Wesentlichen die Form einer Halbkugel. In vorteilhaften Ausführungsformen des erfindungsgemäßen Verfahrens erzeugt man abgerundete Ausnehmungen durch isotropes Ätzen. Bevorzugte Ätzmittel für ein isotropes Ätzen sind ausgewählt aus der Gruppe umfassend ClF₃, BrF₃, XeF₂ und/oder SF₆. Geeignet sind weiterhin andere Silizium isotrop ätzende Medien beispielsweise Mischungen von HNO₃ mit H₂O und NH₄F und/oder Mischungen der vorgenannten Ätzmittel.

Von Vorteil ist, dass Ausführungsformen mit abgerundete Ausnehmungen, insbesondere in Form einer Halbkugel oder im Wesentlichen in Form einer Halbkugel, jeweils eine Teilstruktur poröser Mikrostrukturen definieren können, die eine runde Form aufweisen.

Die Dimensionen der porösen Mikrostrukturen können somit in vorteilhafter Weise bei der Herstellung der Mikrostrukturen definiert werden.

In weiteren bevorzugten Ausführungsformen des erfindungsgemäßen Verfahrens erzeugt man durch anisotropes Ätzen spitze oder eckige Ausnehmungen. Bevorzugte anisotrope Ätzverfahren sind beispielsweise DRIE-(Deep Reactive Ion Etching) Verfahren oder Ätzverfahren unter Verwendung von KOH- und/oder
Tetramethylamoniumhydroxidlösung (TMAH)-Lösungen.

Der mittlere Durchmesser der erzeugten Ausnehmungen liegt in bevorzugten Ausführungsformen im Bereich von ≥ 0,1 µm bis ≤ 500 µm, vorzugsweise im Bereich von ≥ 0,1 µm bis ≤ 100 µm, bevorzugt im Bereich von ≥ 1 µm bis ≤ 50 µm.

Die Tiefe der Ausnehmungen kann beispielsweise im Bereich von wenigen µm bis zu mehreren 100 µm liegen, entsprechend dem Verwendungszweck der Mikrostrukturen. Die Tiefe der Ausnehmungen kann insbesondere bei Ausnehmungen abgerundeter Form, etwa die Hälfte des Durchmessers der Ausnehmungen betragen. In anderen Ausführungsformen kann die Tiefe der Ausnehmungen im Bereich des Durchmessers der Ausnehmungen liegen oder tiefer sein, beispielsweise im Bereich der doppelten bis fünffachen Länge. Dies ist insbesondere vorteilhaft, wenn Mikrostrukturen mit einer Spitze, beispielsweise in Form von Mikronadeln erzeugt werden sollen. In bevorzugten Ausführungsformen des erfindungsgemäßen Verfahrens erzeugt man Ausnehmungen mit einer Tiefe im Bereich von ≥ 1 µm bis ≤ 500 µm, vorzugsweise im Bereich von ≥ 1 µm bis ≤ 100 µm, bevorzugt im Bereich von ≥ 1 µm bis ≤ 50 µm.

In einem folgenden Verfahrensschritt wird eine Opferschicht aufgebracht und strukturiert, wobei die Opferschicht diskrete durchgehende Löcher mit einem Durchmesser im Bereich von ≥ 0,1 µm bis ≤ 150 µm aufweist, die jeweils zentral in den Ausnehmungen angeordnet sind.

Vorzugsweise umfasst die Opferschicht eine Siliziumoxid-Schicht, bevorzugt ist die Opferschicht aus SiO₂ ausgebildet. Beispielsweise kann eine Siliziumoxidschicht durch PECVD (Plasma-Enhanced Chemical Vapor Deposition)-Verfahren aufgebracht werden. Vorzugsweise wird eine Siliziumoxidschicht durch thermische Oxidation erzeugt. Durch thermische Oxidation kann auf dem Si-Halbleitersubstrat ein thermisches Oxid gewachsen werden, das vorzugsweise aus Siliziumoxid ausgebildet ist.

Die Dicke der Opferschicht liegt bevorzugt im Bereich von ≥ 100 nm bis ≤ 5 µm, vorzugsweise im Bereich von ≥ 0,5 µm bis ≤ 4 µm, besonders bevorzugt im Bereich von ≥ 0,5 µm bis ≤ 2 µm.

Die Opferschicht wird vorzugsweise photolithographisch strukturiert. Das Ätzen der Opferschicht erfolgt vorzugsweise mittels gepufferter Oxid-Ätzung (BOE, "buffered oxid etch"), bevorzugt unter Verwendung von mit Ammoniumfluorid gepufferter Flusssäure insbesondere einer HF/NH₄F/H₂O-Lösung. Von Vorteil bei einer gepufferten Oxid-Ätzung ist, dass die Opferschicht entfernbar ist, ohne die darunter liegenden Siliziumschichten zu beschädigen.

In bevorzugten Ausführungsformen des erfindungsgemäßen Verfahrens weisen die diskreten durchgehenden Löcher der Opferschicht einen Durchmesser im Bereich von ≥ 0,1 µm bis ≤ 100 µm, bevorzugt im Bereich von ≥ 0,1 µm bis ≤ 50 µm, weiter bevorzugt im Bereich von ≥ 0,5 µm bis ≤ 50 µm vorzugsweise im Bereich von ≥ 1 µm bis ≤ 20 µm, besonderes bevorzugt im Bereich von ≥ 1 µm bis ≤ 10 µm, auf.

Vorteilhafter Weise ermöglichen die diskreten durchgehenden Löcher der Opferschicht, dass in einem späteren Schritt des Porosifizierens der Strom durch die Löcher der Opferschicht fließen kann. Dies stellt den Vorteil zur Verfügung, dass der Strom vorrangig zu den Zielstrukturen geleitet werden kann und diese bevorzugt porös geätzt werden können. Ein weiterer großer Vorteil, der durch die Löcher in der Opferschicht zur Verfügung gestellt wird, ist, dass diese im weiteren Verlauf des Verfahrens durch Silizium aufgefüllt werden und als Stütz- und/oder Sollbruchstelle der Mikrostrukturen auf dem Halbleitersubstrat dienen können.

In einem folgenden Verfahrensschritt wird auf die Opferschicht Silizium abgeschieden. Hierbei wird Silizium in die Ausnehmungen abgeschieden und eine Siliziumschicht aufgebracht. Die Ausnehmungen werden vorzugsweise vollständig durch das abgeschiedene Silizium aufgefüllt. Weiterhin wird darüber eine vorzugsweise zusammenhängende Siliziumschicht aufgebracht. Geeignet sind sowohl polykristalline als auch einkristalline Schichten, wobei eine polykristalline Schicht bevorzugt ist. Vorzugsweise kann eine polykristalline Siliziumschicht durch PECVD (Plasma-Enhanced Chemical Vapor Deposition)-Verfahren aufgebracht werden. Weiterhin kann eine Siliziumschicht durch PECVD-Verfahren aufgebracht werden, auf die eine epitaktisch abgeschiedene polykristalline Silizium-Schicht aufgebracht werden kann.

In bevorzugten Ausführungsformen des Verfahrens bringt man eine Siliziumschicht mit einer Dicke oberhalb der Ausnehmungen im Bereich von ≥ 1 µm bis ≤ 100 µm, vorzugsweise im Bereich von ≥ 2 µm bis ≤ 50 µm, bevorzugt im Bereich von ≥ 5 µm bis ≤ 20 µm, auf.

Die Dicke der Siliziumschicht oberhalb der Ausnehmungen kann insbesondere bei herzustellenden Mikrostrukturen mit runder oder im Wesentlichen runder Form, der Tiefe der Ausnehmungen und/oder etwa der Hälfte des Durchmessers der Ausnehmungen entsprechen. In anderen Ausführungsformen kann die Dicke der Siliziumschicht im Bereich des Durchmessers der Ausnehmungen liegen oder geringer sein. Dies ist insbesondere vorteilhaft, wenn Mikrostrukturen in Form von Mikronadeln erzeugt werden sollen.

In bevorzugten Ausführungsformen des Verfahrens wird die Siliziumschicht planarisiert und/oder dotiert.

In bevorzugten Ausführungsformen kann die Siliziumschicht planarisiert werden. Ein bevorzugtes Verfahren des Planarisierens ist das Polieren. In bevorzugten Ausführungsformen kann die Siliziumschicht poliert werden, vorzugsweise mittels CMP (chemisch-mechanischem Polieren), bei welchem die abrasive Wirkung eines gekörnten Polierkopfes durch die chemisch Wirkung einer passenden Lösung unterstützt wird. Durch das Planarisieren insbesondere Polieren lässt sich die Oberfläche der Mikrostrukturen definieren und im folgenden definiert formen, da die Topographie, welche durch das Aufbringen der Siliziumschicht auf die Ausnehmungen entstanden ist, durch diesen Schritt nivelliert werden kann.

In weiter bevorzugten Ausführungsformen kann die Siliziumschicht dotiert werden. Eine Dotierung erfolgt vorzugsweise mit Bor, insbesondere kann man mit Bor implantieren oder eine Borglasbelegung vornehmen. Es kann bevorzugt sein, dass die aufgebrachte Siliziumschicht dotiert wird. In anderen Ausführungsformen kann es bevorzugt sein, die Dotierung beim Aufbringen der Siliziumschicht in das Material einzubringen.

Durch die Wahl der Dotierung kann vorteilhafter Weise die Porenstruktur beeinflusst werden. Es kann vorgesehen sein, eine Dotierung im Bereich von bis zu 10¹⁹/cm³ zu verwenden, wobei die Angabe der Zahl der Dotieratome pro cm³ des Si-Halbleitersubstrats entspricht. Durch eine Dotierung mit 10¹⁹/cm³ kann eine mesoporöse Struktur erzielt werden, deren Porendurchmesser vorzugsweise im Bereich von ≥ 2 nm bis ≤ 100 nm liegt. Der Vorteil einer mesoporösen Struktur der Porosität der Mikrostrukturen liegt insbesondere darin, dass Substanzen oder Wirkstoffe, die beispielsweise in einen Körper eingebracht werden sollen, gut in mesoporöse Strukturen eingebracht werden können.

Bevorzugt wird in flusssäurehaltigen Elektrolyten insbesondere wässrigen Flusssäurelösungen, oder Gemischen enthaltend Flusssäure, Wasser und weitere Reagenzien, insbesondere ausgewählt aus der Gruppe umfassend Netzmittel beispielsweise Alkohole, vorzugsweise ausgewählt aus der Gruppe umfassend Ethanol und/oder Isopropanol, und/oder Entspannungsmittel beispielsweise Tenside porosifiziert. Beispielsweise sind Ethanol und/oder Isopropanol im Bereich von ≥ 10 Vol.-% bis ≤ 30 Vol.-%, bezogen auf das Gesamtvolumen des Elektrolyten, verwendbar.

Bevorzugt liegt der Flusssäure-Gehalt einer wässrigen Flusssäure-Lösung, im Bereich von ≥ 10 Vol.-% bis ≤ 40 Vol.-%, bezogen auf das Gesamtvolumen des Elektrolyten. Es kann ein Netzmittel hinzu gegeben werden. Bevorzugte Netzmittel sind ausgewählt aus der Gruppe umfassend Isopropanol und/oder Ethanol. Bevorzugte Stromdichten liegen im Bereich von ≥ 10 mA/cm² bis ≤ 200 mA/cm², vorzugsweise im Bereich von zwischen ≥ 50 mA/cm² bis ≤ 150 mA/cm².

Ein besonderer Vorteil des erfindungsgemäßen Verfahrens kann dadurch zur Verfügung gestellt werden, dass der Strom vorzugsweise durch die Löcher in der Opferschicht fließen kann und vorrangig zu den herzustellenden Strukturen geleitet wird. Hierdurch werden diese bevorzugt porös geätzt.

Die Porosität ist durch geeignete Wahl der Prozessparameter, beispielsweise der Elektrolytzusammensetzung insbesondere der Flusssäurekonzentration und/oder der Stromdichte einstellbar. Die Porosität gibt das Verhältnis des Leerraums innerhalb einer Struktur und des verbleibenden Halbleitersubstrat- oder Siliziummaterial an. So ist vorzugsweise eine Porosität der porösen Mikrostrukturen in einem Bereich von ca. 10 % bis über 90% einstellbar, bevorzugt im Bereich von ≥ 10 % bis ≤ 80 %, besonders bevorzugt im Bereich von ≥ 20 % bis ≤ 70 %.

In bevorzugten Ausführungsformen des Verfahrens stellt man eine Porosität der porösen Mikrostrukturen in einem Bereich von ≥ 10 % bis ≤ 90% ein.

"Porosität" wird im Sinne der vorliegenden Erfindung so definiert, dass sie den Leerraum innerhalb der Struktur und des verbleibenden Substratmaterials angibt. Sie kann entweder optisch bestimmt werden, also aus der Auswertung beispielsweise von Mikroskopaufnahmen, oder chemisch. Im Falle der chemischen Bestimmung gilt: Porosität P = (m1-m2)/(m1-m3), wobei ml die Masse der Probe vor dem Porosifizieren ist, m2 die Masse der Probe nach dem Porosifizieren und m3 die Masse der Probe nach Ätzen mit 1 molarer NaOH-Lösung, welches die poröse Struktur chemisch auflöst. Alternativ kann die poröse Struktur auch durch eine KOH/Isopropanol-Lösung aufgelöst werden.

Je nach Prozessparameter sind verschiedene Porenstrukturen erzeugbar, so können Nano-, Meso- oder Makroporen erzeugt werden. Die Porengröße kann je nach Flusssäurekonzentration, Dotierung und Stromdichte in einem Bereich von einigen Nanometern bis 50 nm Durchmesser eingestellt werden. Beispielsweise sind Poren mit einem Durchmesser ≤ 5 nm, im Bereich von zwischen ≥ 5 nm bis ≤ 50 nm, oder ≥ 50 nm herstellbar. In bevorzugten Ausführungsformen sind Poren mit einem Durchmesser im Bereich von ≥ 1 nm bis ≤ 1 µm, vorzugsweise im Bereich von ≥ 2 nm bis ≤ 100 nm, bevorzugt im Bereich von ≥ 5 nm bis ≤ 30 nm herstellbar.

In einem weiteren Verfahrensschritt erfolgt ein Aufbringen und Strukturieren einer weiteren Maskierungsschicht auf die porosifizierte Silizium-Schicht, wobei die weitere Maskierungsschicht die Bereiche oberhalb der Ausnehmungen bedeckt, die eine weitere Teilstruktur der porösen Mikrostrukturen ausbilden. Hierdurch kann in vorteilhafter Weise die laterale Struktur der herzustellenden Mikropartikel ausgebildet werden.

Vorzugsweise verwendet man hierfür eine Photoresistschicht mit positiven oder negativen Belichtungseigenschaften, die anschließend bevorzugt mittels photolithographischer Verfahren strukturiert wird. Geeignet sind beispielsweise flüssige Resistlacke wie Photolack. Die Photoresistschicht kann mit einer Maske, beispielsweise einer Chrom-Schicht auf einem Quarz-Substrat, belichtet werden.

Die Bereiche oberhalb der Ausnehmungen, die die Maskierungsschicht bedeckt, weisen vorzugsweise einen dem Durchmesser der Ausnehmungen entsprechenden Durchmesser und/oder eine der Form der Ausnehmungen entsprechende Form auf.

In einem weiteren Verfahrensschritt wird die porosifizierte Silizium-Schicht anisotrop geätzt. Hierbei verbleiben die durch die Maskierungsschicht geschützten Bereiche der porosifizierten Siliziumschicht oberhalb der Ausnehmungen. Die nach dem Ätzen verbleibenden Strukturen bilden eine weitere Teilstruktur der porösen Mikrostrukturen aus. Die unterhalb der porosifizierte Silizium-Schicht liegende Opferschicht wird durch das anisotrope Ätzen nicht geätzt. Bevorzugte Verfahren sind insbesondere DRIE-(Deep Reactive Ion Etching) Verfahren. Ein bevorzugtes Ätzmittel ist hierbei SF₆.

Nach dem Ätzen kann die Maskierungsschicht entfernt werden.

In einem optionalen Verfahrensschritt können die durch die Maskierungsschicht geschützten Bereiche der porosifizierten Siliziumschicht oberhalb der Ausnehmungen, die die weitere Teilstruktur der porösen Mikrostrukturen ausbilden, abgerundet werden, vorzugsweise durch isotropes Ätzen.

In bevorzugten Ausführungsformen des Verfahrens rundet man die aus der Opferschicht herausragende Teilstruktur der porösen Mikrostrukturen durch isotropes Ätzen ab.

In bevorzugten Ausführungsformen kann das Abrunden durch ein kurzes Eintauchen in eine KOH-, und/oder eine so genannte HNA-Lösung umfassend HF, vorzugsweise 1 Vol.%, HNO₃, vorzugsweise 3 Vol.%, und CH₃COOH, vorzugsweise 8 Vol.%, jeweils bezogen auf das Gesamtvolumen, erfolgen. Eine weitere bevorzugte Ausführungsform sieht das Einbringen der Mikrostrukturen in eine ClF₃-haltige Atmosphäre vor. Insbesondere ist von Vorteil, dass durch ein Abrunden der Strukturen oberhalb der Ausnehmungen runde oder im Wesentlichen runde poröse Silizium-Mikrostrukturen herstellbar sind. Es kann jedoch auch im Fall von herzustellenden poröse Mikrostrukturen in Form von Mikronadeln von Vorteil sein, die Struktur abzurunden.

In einem weiteren Verfahrensschritt erfolgt ein Entfernen der Opferschicht. Vorzugsweise kann das Entfernen durch Ätzen in Flusssäure-(HF)Dampf erfolgen. Dies kann den Vorteil zur Verfügung stellen, dass die porösen Mikrostrukturen durch die Siliziumbereiche innerhalb der vormaligen Löcher der Opferschicht auf dem Si-Halbleitersubstrat gehalten werden. Dies ermöglicht, dass die Mikrostrukturen in einem optionalen Verfahrensschritt auf dem Halbleitersubstrat funktionalisiert werden können.

In anderen bevorzugten Ausführungsformen kann die Entfernung der Opferschicht durch Ätzen in Flusssäure-(HF)Lösung erfolgen. Dies kann der Vorteil zur Verfügung stellen, dass die porösen Mikrostrukturen durch das Ätzen von dem Si- Halbleitersubstrat abgelöst werden. Die porösen Mikrostrukturen können beispielsweise durch Filtern aus der Lösung gewonnen werden.

In weiteren Ausführungsformen mit geeigneten Opferschichten, beispielsweise ein Schichtsystem bestehend aus Oxid und Silizium-Germanium-Schichten, kann die Opferschicht durch Gasphasenätzen mittels Ätzmittel beispielsweise ausgewählt aus der Gruppe umfassend ClF₃ und/oder XeF₂ entfernt werden.

In bevorzugten Ausführungsformen des Verfahrens funktionalisiert man die porösen Mikrostrukturen vor dem Abtrennen von dem Halbleitersubstrat. Beispielsweise können die porösen Mikrostrukturen auf dem Halbleitersubstrat dotiert, imprägniert, beladen oder oxidiert werden, bevor sie abgetrennt werden. Vorzugsweise werden die porösen Mikrostrukturen mit Wirksubstanzen beispielsweise Arzneistoffen imprägniert. Dies stellt den Vorteil zur Verfügung, dass eine einheitliche und kontrollierte Funktionalisierung auf dem Halbleitersubstrat erfolgen kann.

Bevorzugt werden die porösen Mikrostrukturen durch mechanische Einwirkung, Ultraschall oder durch Überströmen eines Druckgases abgetrennt oder vereinzelt. Von Vorteil ist hierbei, dass die porösen Mikrostrukturen, die durch Stützstellen gehalten werden, durch geringe mechanische Einwirkung abgelöst werden können.

Von besonderem Vorteil ist hierbei, dass das Halbleitersubstrat insbesondere ein Siliziumwafer hierdurch nicht völlig zerstört werden muss, sondern erneut verwendet werden kann. Beispielsweise kann das Halbleitersubstrat nach Schleifen und/oder Polieren erneut zur Herstellung von porösen Mikrostrukturen verwendbar sein. Das erfindungsgemäße Verfahren ist weiterhin vorteilhaft insofern, als die erneute Verwendbarkeit des Halbleitersubstrats ermöglicht, die Herstellungskosten poröser Mikrostrukturen aus Silizium zu senken.

Ein weiterer Gegenstand der vorliegenden Erfindung betrifft poröse Mikrostrukturen, die gemäß dem erfindungsgemäßen Verfahren hergestellt sind.

Grundsätzlich sind die porösen Mikrostrukturen für alle Anwendungen geeignet, die poröse Mikrostrukturen insbesondere poröse Partikel oder Mikronadeln erfordern. Insbesondere sind die Mikrostrukturen für biologische Anwendungen geeignet, da Mikrostrukturen aus porösem Silizium biokompatibel sind und insbesondere vom Organismus resorbiert werden können. In bevorzugten Verwendungen der porösen Mikrostrukturen können diese als Reservoir für die Applikation von Wirkstoffen oder aktiven Substanzen verwendet werden oder zur Herstellung von Applikationseinheiten für Wirkstoffe oder Medikamente dienen. Von Vorteil ist, dass die porösen Mikrostrukturen als Reservoir für Wirkstoffe oder aktive Substanzen verwendbar sind. Weiterhin können die porösen Mikrostrukturen eine schmerzfreie Applikation von Wirkstoffen oder Medikamenten ermöglichen.

Vorteilhaft ist weiterhin eine Verwendung der erfindungsgemäß hergestellten porösen Mikrostrukturen zur Einspritzung in die Blutbahn.

Eine bevorzugte Verwendung ist eine lokalisierte Applikation von Medikamenten. Beispielsweise sind mit einem Angiogeneseinhibitor beladene poröse Mikrostrukturen geeignet zum Verstopfen von Blutgefässen, die einen Tumor versorgen. Von Vorteil ist hierbei, dass durch die Freisetzung der Substanz die mechanische Verstopfungswirkung durch die anti-angiogenetische Wirkung lokal unterstützt werden kann.

Beispielsweise verwendbare anti-angiogenetische Substanzen sind ausgewählt aus der Gruppe umfassend Bevacizumab erhältlich unter dem Handelsnamen Avastin® bei der Firma Genentech/Roche, Vandetanib (ZD6474) erhältlich unter dem Handelsnamen Zactima® bei der FirmaAstra-Zeneca Plc. und/oder die Substanz PTK787/ZK222584 (Novartis/Schering).

Die porösen Mikrostrukturen können eine beliebige Form aufweisen. Vorzugsweise sind die hergestellten porösen Mikrostrukturen rund oder im Wesentlichen rund oder weisen die Form einer Mikronadel auf.

Ein besonderer Vorteil des erfindungsgemäßen Verfahrens kann dadurch zur Verfügung gestellt werden, dass runde oder im Wesentlichen runde poröse Silizium-Mikrostrukturen herstellbar sind. In bevorzugten Ausführungsformen sind runde oder im Wesentlichen runde poröse Silizium-Mikrostrukturen herstellbar, die einen Durchmesser im Bereich von ≥ 1 µm bis ≤ 500 µm, vorzugsweise im Bereich von ≥ 1 µm bis ≤ 50 µm, besonders bevorzugt im Bereich von ≥ 1 um bis ≤ 5 µm aufweisen. Diese porösen Silizium-Mikrostrukturen sind insbesondere verwendbar für eine Einspritzung in die Blutbahn. Von Vorteil hierbei ist, dass runde poröse Silizium-Mikrostrukturen mit einem Durchmesser kleiner als 5 µm dazu führen können, dass keine Gefäße verschlossen werden, oder nur in sehr geringem Ausmaß.

In weiteren bevorzugten Ausführungsformen sind runde oder im Wesentlichen runde poröse Silizium-Mikrostrukturen herstellbar, die einen Durchmesser im bereich von ≥ 1 µm bis ≤ 500 µm, vorzugsweise im Bereich von ≥ 1 µm bis ≤ 50 µm, besonders bevorzugt im Bereich von ≥ 10 µm bis ≤ 50 µm aufweisen. Diese porösen Silizium-Mikrostrukturen können ermöglichen, dass sich Gefäße gezielt verschließen lassen. Weiterhin kann sich eine Akkumulation der porösen Silizium-Mikrostrukturen erzielen lassen. Eine mögliche Anwendungen ist beispielsweise eine Verabreichung radioaktiver Tracer, die sich in Organen sammeln und so eine Untersuchung der Durchblutung ermöglichen. Eine weitere denkbare Anwendung ist die gezielte Unterbindung des Blutflusses beispielsweise in der Onkologie.

Die durch das erfindungemäße Verfahren hergestellten porösen Mikrostrukturen weisen vorzugsweise eine typische Orientierung der Poren auf. Die in den porösen Mikrostrukturen von der Substratoberfläche ausgehenden Poren sind vorzugsweise senkrecht zu den Poren orientiert, die von den Ausnehmungen ausgehen.

## Patentansprüche

1. Verfahren zur Herstellung poröser Mikrostrukturen (8, 8') in einem Si-Halbleitersubstrat (1) umfassend die folgenden Schritte:
a) Aufbringen und Strukturieren einer Maskierungsschicht auf die äußere Oberfläche der Vorderseite eines Si-Halbleitersubstrats (1), wobei in der Maskierungsschicht diskrete Löcher mit einem mittleren Durchmesser im Bereich von ≥ 0,1 µm bis ≤ 500 µm ausgebildet werden,
b) Erzeugen von Ausnehmungen (2, 2') in einem Si-Halbleitersubstrat (1), wobei die Ausnehmungen (2, 2') jeweils eine Teilstruktur der porösen Mikrostrukturen (8, 8') definieren;
c) Entfernen der Maskierungsschicht;
d) Aufbringen und Strukturieren einer Opferschicht (3), wobei die Opferschicht (3) diskrete durchgehende Löcher (4, 4') mit einem Durchmesser im Bereich von ≥ 0,1 µm bis ≤ 150 µm aufweist, die jeweils zentral in den Ausnehmungen (2, 2') angeordnet sind;
e) Aufbringen einer Siliziumschicht (5), wobei die Siliziumschicht (5) die Ausnehmungen (2, 2') auffüllt und eine Schicht oberhalb der Opferschicht (3) ausbildet;
f) Porosifizieren der Silizium-Schicht (5);
g) Aufbringen und Strukturieren einer weiteren Maskierungsschicht auf die porosifizierte Silizium-Schicht (5), wobei die weitere Maskierungsschicht die Bereiche oberhalb der Ausnehmungen (2, 2') bedeckt, die eine weitere Teilstruktur der porösen Mikrostrukturen (8, 8') ausbilden;
h) anisotropes Ätzen der porosifizierten Silizium-Schicht (5);
i) Entfernen der weiteren Maskierungsschicht;
j) Entfernen der Opferschicht (3);
k) Abtrennen oder Vereinzeln der porösen Mikrostrukturen (8, 8').

2. Verfahren nach Anspruch 1, **dadurch gekennzeichnet, dass** man durch isotropes Ätzen abgerundete Ausnehmungen (2') erzeugt.

3. Verfahren nach Anspruch 1 oder 2, **dadurch gekennzeichnet, dass** man durch anisotropes Ätzen spitze oder eckige Ausnehmungen (2) erzeugt.

4. Verfahren nach einem der vorherigen Ansprüche, **dadurch gekennzeichnet, dass** man Ausnehmungen (2, 2') mit einer Tiefe im Bereich von ≥ 1 µm bis ≤ 500 µm, vorzugsweise im Bereich von ≥ 1 µm bis ≤ 100 µm, bevorzugt im Bereich von ≥ 1 µm bis ≤ 50 µm, erzeugt.

5. Verfahren nach einem der vorherigen Ansprüche, **dadurch gekennzeichnet, dass** die diskreten durchgehenden Löcher (4, 4') in der Opferschicht (3) einen Durchmesser im Bereich von ≥ 0,1 µm bis ≤ 50 µm, bevorzugt im Bereich von ≥ 1 µm bis ≤ 20 µm aufweisen.

6. Verfahren nach einem der vorherigen Ansprüche, **dadurch gekennzeichnet, dass** man in Schritt e) eine Siliziumschicht (5) mit einer Dicke oberhalb der Ausnehmungen (2, 2') im Bereich von ≥ 1 µm bis ≤ 100 µm, vorzugsweise im Bereich von ≥ 2 µm bis ≤ 50 µm, bevorzugt im Bereich von ≥ 5 µm bis ≤ 20 µm, aufbringt.

7. Verfahren nach einem der vorherigen Ansprüche, **dadurch gekennzeichnet, dass** man die aus der Opferschicht (3) herausragende Teilstruktur (7) der porösen Mikrostrukturen (8, 8') durch isotropes Ätzen abrundet.

8. Verfahren nach einem der vorherigen Ansprüche, **dadurch gekennzeichnet, dass** man die Siliziumschicht (5) planarisiert und/oder dotiert.

9. Verfahren nach einem der vorherigen Ansprüche, **dadurch gekennzeichnet, dass** man eine Porosität der porösen Mikrostrukturen (8, 8') in einem Bereich von ≥ 10 % bis ≤ 90% einstellt.

10. Verfahren nach einem der vorherigen Ansprüche, **dadurch gekennzeichnet, dass** man die porösen Mikrostrukturen (8, 8') vor dem Abtrennen funktionalisiert.

## Claims

1. Method for producing porous microstructures (8, 8') in an Si semiconductor substrate (1) comprising the following steps:
a) applying a masking layer to the outer surface of the front side of an Si semiconductor substrate (1) and patterning said masking layer, wherein discrete holes having an average diameter in the range of ≥ 0.1 µm to ≤ 500 µm are formed in the masking layer,
b) producing cutouts (2, 2') in an Si semiconductor substrate (1), wherein the cutouts (2, 2') in each case define a partial structure of the porous microstructures (8, 8');
c) removing the masking layer;
d) applying and patterning a sacrificial layer (3), wherein the sacrificial layer (3) has discrete continuous holes (4, 4') having a diameter in the range of ≥ 0.1 µm to ≤ 150 µm, which are in each case arranged centrally in the cutouts (2, 2');
e) applying a silicon layer (5), wherein the silicon layer (5) fills the cutouts (2, 2') and forms a layer above the sacrificial layer (3),
f) porosifying the silicon layer (5);
g) applying a further masking layer to the porisified silicon layer (5) and patterning said further masking layer, wherein the further masking layer covers the regions above the cutouts (2, 2') which form a further partial structure of the porous microstructures (8, 8');
h) anistropically etching the porisified silicon layer (5);
i) removing the further masking layer;
j) removing the sacrificial layer (3);
k) separating or singulating the porous microstructures (8, 8').

2. Method according to Claim 1, **characterized in that** rounded cutouts (2') are produced by isotropic etching.

3. Method according to Claim 1 or 2, **characterized in that** pointed or angular cutouts (2) are produced by anisotropic etching.

4. Method according to any of the preceding claims, **characterized in that** cutouts (2, 2') having a depth in the range of ≥ 1 µm to ≤ 500 µm, preferably in the range of ≥ 1 µm to ≤ 100 µm, preferably in the range of ≥ 1 µm to ≤ 50 µm, are produced.

5. Method according to any of the preceding claims, **characterized in that** the discrete continuous holes (4, 4') in the sacrificial layer (3) have a diameter in the range of ≥ 0.1 µm to ≤ 50 µm, preferably in the range of ≥ 1 µm to ≤ 20 µm.

6. Method according to any of the preceding claims, **characterized in that** step e) involves applying a silicon layer (5) having a thickness above the cutouts (2, 2') in the range of ≥ 1 µm to ≤ 100 µm, preferably in the range of ≥ 2 µm to ≤ 50 µm, preferably in the range of ≥ 5 µm to ≤ 20 µm.

7. Method according to any of the preceding claims, **characterized in that** the partial structure (7) of the porous microstructures (8, 8') which projects from the sacrificial layer (3) is rounded by isotropic etching.

8. Method according to any of the preceding claims, **characterized in that** the silicon layer (5) is planarized and/or doped.

9. Method according to any of the preceding claims, **characterized in that** a porosity of the porous microstructures (8, 8') in a range of ≥ 10% to ≤ 90% is set.

10. Method according to any of the preceding claims, **characterized in that** the porous microstructure (8, 8') are functionalized before separation.

## Revendications

1. Procédé de fabrication de microstructures poreuses (8, 8') dans un substrat semi-conducteur (1), le procédé comportant les étapes qui consistent à :
a) placer et structurer une couche de masquage sur la surface extérieure de la face avant d'un substrat semi-conducteur (1) en Si, des trous distincts d'un diamètre moyen compris dans la plage de ≥ 0,1 µm à ≤ 500 µm étant formés dans la couche de masquage,
b) former des découpes (2, 2') dans un substrat semi-conducteur (1) en Si, chacune des découpes (2, 2') définissant des microstructures poreuses (8, 8'),
c) enlever la couche de masquage,
d) placer et structurer une couche sacrifiée (3), la couche sacrifiée (3) présentant des perforations (4, 4') dont le diamètre est compris dans la plage de ≥ 0,1 µm à ≤ 150 µm et toutes disposées au centre des découpes (2, 2'),
e) appliquer une couche de silicium (5), la couche de silicium (5) remplissant les découpes (2, 2') et formant une couche au-dessus de la couche sacrifiée (3),
f) rendre poreuse la couche de silicium (5),
g) placer et structurer une autre couche de masquage sur la couche de silicium (5) rendue poreuse, l'autre couche de masquage recouvrant les parties situées au-dessus des découpes (2, 2') et formant une autre partie des microstructures poreuses (8, 8'),
h) graver de manière anisotrope la couche de silicium (5) rendue poreuse,
i) enlever l'autre couche de masquage,
j) enlever la couche sacrifiée (3) et
k) découper ou séparer une à une les microstructures poreuses (8, 8').

2. Procédé selon la revendication 1, **caractérisé en ce que** l'on forme par gravure isotrope des découpes (2') arrondies.

3. Procédé selon les revendications 1 ou 2, **caractérisé en ce que** l'on forme par gravure anisotrope des découpes (2) pointues ou anguleuses.

4. Procédé selon l'une des revendications précédentes, **caractérisé en ce que** l'on forme des découpes (2, 2') d'une profondeur comprise dans la plage de ≥ 1 µm à ≤ 500 µm, de préférence dans la plage de ≥ 1 µm à ≤ 100 µm et mieux dans la plage de ≥ 1µm à ≤ 50 µm.

5. Procédé selon l'une des revendications précédentes, **caractérisé en ce que** les perforations distinctes (4, 4') ménagées dans la couche sacrifiée (3) ont un diamètre compris dans la plage de ≥ 0,1 µm à ≤ 50 µm et de préférence dans la plage de ≥ 1 µm à ≤ 20 µm.

6. Procédé selon l'une des revendications précédentes, **caractérisé en ce que** dans l'étape e), on applique une couche de silicium (5) dont l'épaisseur au-dessus des découpes (2, 2') est comprise dans la plage de ≥ 1 µm à ≤ 100 µm, de préférence dans la plage de ≥ 2 µm à ≤ 50 µm et mieux dans la plage de ≥ 5 µm à ≤ 20 µm.

7. Procédé selon l'une des revendications précédentes, **caractérisé en ce que** la partie (7) des microstructures poreuses (8, 8') qui déborde hors de la couche sacrifiée (3) est arrondie par gravure isotrope.

8. Procédé selon l'une des revendications précédentes, **caractérisé en ce que** la couche de silicium (5) est planée et/ou dopée.

9. Procédé selon l'une des revendications précédentes, **caractérisé en ce que** l'on établit pour les microstructures poreuses (8, 8') une porosité comprise dans la plage de ≥ 10 % à ≤ 90 %.

10. Procédé selon l'une des revendications précédentes, **caractérisé en ce qu'**avant la découpe, les microstructures poreuses (8, 8') sont fonctionnalisées.
